(19) Europäisches Patentamt
European Patent Office
Office européen des brevets

(11) **EP 1 424 996 B1**

(12) **EUROPÄISCHE PATENTSCHRIFT**

(45) Veröffentlichungstag und Bekanntmachung des Hinweises auf die Patenterteilung:
**27.04.2005 Patentblatt 2005/17**

(21) Anmeldenummer: **02796206.7**

(22) Anmeldetag: **03.08.2002**

(51) Int Cl.⁷: **A61K 9/14**, A61K 31/46,
A61P 11/06, A61K 9/72

(86) Internationale Anmeldenummer:
**PCT/EP2002/008692**

(87) Internationale Veröffentlichungsnummer:
**WO 2003/017979 (06.03.2003 Gazette 2003/10)**

(54) **VERFAHREN ZUR HERSTELLUNG VON INHALATIONSPULVERN**

METHOD FOR THE PRODUCTION OF INHALATION POWDERS

PROCEDE DE PRODUCTION DE POUDRES POUR INHALATION

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **23.08.2001 DE 10141376**

(43) Veröffentlichungstag der Anmeldung:
**09.06.2004 Patentblatt 2004/24**

(73) Patentinhaber: **Boehringer Ingelheim Pharma GmbH & Co.KG**
**55216 Ingelheim am Rhein (DE)**

(72) Erfinder:
• **BOECK, Georg**
**55122 MAINZ (DE)**
• **WALZ, Michael**
**55411 BINGEN AM RHEIN (DE)**

(56) Entgegenhaltungen:
**WO-A-02/30390**

**Beschreibung**

Hintergrund der Erfindung

Bei der Behandlung einer Vielzahl von Erkrankungen, insbesondere bei der Behandlung von Atemwegserkrankungen bietet sich die inhalative Applikation des Wirkstoffs an. Neben der inhalativen Applikation therapeutisch wirksamer Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt.

In der Pulvermischtechnologie werden üblicherweise Mischverfahren eingesetzt, die auf der Verdünnungsmethode basieren. Hierbei wird der gesamte Wirkstoff vorgelegt und anschließend mit Hilfsstoff beispielsweise in Verhältnissen von 1:1, 1:2 oder 1:4 versetzt und gemischt. Zu den so erhaltenen Mischungen wird dann erneut Hilfsstoff in vergleichbarem Verhältnis zugesetzt. Dieses Vorgehen wird üblicherweise solange wiederholt, bis der ganze Hilfsstoffanteil untergemischt ist. Problematisch an einer solchen Vorgehensweise sind die daraus unter Umständen resultierenden Homogenitätsprobleme. Diese treten insbesondere bei Mischungen auf, bei denen die Substanzen ein stark unterschiedliches Teilchengrößenspektrum aufweisen. Besonders bemerkbar wird dies zudem bei Pulvermischungen, bei denen die Substanz mit der kleineren Teilchengrößenverteilung, der Wirkstoff, nur einen sehr geringen Mengenanteil an der Gesamtpulvermenge aufweist.

Es ist daher Aufgabe der vorliegenden Erfindung, ein Verfahren bereitzustellen, welches die Herstellung von Inhalationspulvern erlaubt, die durch ein hohes Maß an Homogenität im Sinne der Gehaltsgleichförmigkeit gekennzeichnet sind.

Detaillierte Beschreibung der Erfindung

Überraschenderweise wurde gefunden, daß die eingangs genannte Aufgabe durch ein Verfahren gelöst wird, in dem die Substanz mit der kleineren Teilchengrößenverteilung und die Substanz mit der größeren Teilchengrößenverteilung in einen geeigneten Mischbehälter kontinuierlich so eindosiert werden, daß der Quotient N aus der Fördergeschwindigkeit für die Zudosierung der Substanz mit der kleineren Teilchengrößenverteilung und Fördergeschwindigeit für die Zudosierung der Substanz mit der größeren Teilchengrößenverteilung wenigstens so groß ist wie der Quotient M aus Gesamtmasse der Substanz mit der kleineren Teilchengrößenverteilung und Gesamtmasse der Substanz mit der größeren Teilchengrößenverteilung.

Im Rahmen der vorliegenden Erfindung repräsentiert, soweit nicht anders definiert, die Substanz mit der kleineren Teilchengrößenverteilung, die sehr fein gemahlen und in einem sehr geringen Massenanteil in der resultierenden Pulverformulierung vorliegt, den Wirkstoff. Im Rahmen der vorliegenden Erfindung repräsentiert, soweit nicht anders definiert, die Substanz mit der größeren Teilchengrößenverteilung, die grob gemahlen und in einem grossen Massenanteil in der resultierenden Pulverformulierung vorliegt, den Hilfsstoff.

Dementsprechend betrifft ein Aspekt der vorliegenden Erfindung ein Verfahren zur Herstellung von Inhalationspulvern in dem der Wirkstoff und der Hilfsstoff in einen geeigneten Mischbehälter kontinuierlich so eindosiert werden, daß der Quotient N aus der Fördergeschwindigkeit für die Zudosierung des Wirkstoffs und Fördergeschwindigeit für die Zudosierung des Hilfsstoffs wenigstens so groß ist wie der Quotient M aus Gesamtmasse des Wirkstoffs und Gesamtmasse des Hilfsstoffs.

Unter Fördergeschwindigkeit wird im Rahmen der vorliegenden Erfindung der Quotient aus pro Zeitintervall geförderter Masse verstanden (z.B. g/min). Unter Gesamtmasse der jeweiligen Komponenten wird im Rahmen der vorliegenden Erfindung die Gesamtmasse der einzelnen Bestandteile in dem mittels des erfindungsgemäßen Verfahren herstellbaren Inhaltionspulver verstanden.

Der Quotient N kann dementsprechend auch wie folgt dargestellt werden:

$$N = \frac{\text{Fördergeschwindigkeit (Substanz mit kleinerer Teilchengrößenverteilung)}}{\text{Fördergeschwindigkeit (Substanz mit größerer Teilchengrößenverteilung)}}$$

Der Quotient M kann dementsprechend auch wie folgt dargestellt werden:

$$M = \frac{\text{Gesamtmasse (Substanz mit kleinerer Teilchengrößenverteilung)}}{\text{Gesamtmasse (Substanz mit größerer Teilchengrößenverteilung)}}$$

Ist N = M hat dies zur Folge, daß die kontinuierliche Zudosierung der Komponenten gleichzeitig beendet ist, sofern sie gleichzeitig begonnen hat. Erfindungsgemäß bevorzugt ist N > M. Bevorzugt liegt der Quotient aus N/M in einem Bereich von 1 < N/M ≤ 1,5, besonders bevorzugt 1,001 ≤ N/M ≤ 1,2. Verfahren, in denen der Quotient N/M in einem Bereich von 1,01 ≤ N/M ≤ 1,15, bevorzugt 1,02 ≤ N/M ≤ 1,1 liegt, sind erfindungsgemäß von besonderer Bedeutung.

Die Dauer der Zudosierung (Förderung) der beiden Komponenten ist naturgemäß unter anderem abhängig von der gewünschten Gesamtmenge an herzustellender Pulvermischung. Es ist für den Fachmann ersichtlich, daß bei Herstellung von nur kleineren Chargen an Pulvermischung, die Zudosierung über einen kleineren Zeitraum erfolgen kann, als bei größeren Pulvermengen. In der Regel werden die beiden Komponenten beispielsweise im Kilogrammbereich wenigstens über einen Zeitraum von 5 Minuten kontinuierlich zudosiert. Vorzugsweise wird das erfindungsgemäße Verfahren jedoch so durchgeführt, daß die pro Zeitintervall geförderte Menge der jeweiligen Komponenten möglichst gering ist. Fördergeschwindigkeiten von etwa 0,5-15 g/min, vorzugsweise 1-10 g/min Wirkstoff bei Kilogrammchargen (bezogen auf die Gesamtpulvermenge) sind erfindungsgemäß bevorzugt. Fördergeschwindigkeiten von etwa 100 - 2000 g/min, vorzugsweise 500 - 1500 g/min Hilfsstoff bei Kilogrammchargen (bezogen auf die Gesamtpulvermenge) sind erfindungsgemäß bevorzugt.

Bevorzugt erfolgt die Zugabe der Komponenten Wirkstoff und Hilfsstoff im Rahmen des erfindungsgemäßen Verfahrens über eine geeignete Siebeinrichtung, vorzugsweise über einen Siebgranulator mit einer Maschenweite von 0,1 bis 2 mm, besonders bevorzugt 0,3 bis 1 mm, höchst bevorzugt 0,3 bis 0,6 mm.

Erfindungsgemäß bevorzugt wird das Verfahren so durchgeführt, daß zunächst mit der Eindosierung der Hilfsstoffkomponente in den geeigneten Mischbehälter begonnen wird. Kurz nach Beginn der Förderung des Hilfsstoffs wird dann mit der kontinuierlichen Förderung (Zudosierung) des Wirkstoffs begonnen. Besonders bevorzugt werden die Fördergeschwindigkeiten von Hilfsstoff und Wirkstoff dabei so aufeinander abgestimmt, daß nach vollständiger Zugabe des Wirkstoffs die Förderung des Hilfsstoffs noch nicht vollständig abgeschlossen ist. In diesem Fall ist der Quotient N/M >1. Durch den Vorlauf bei der Förderung des Hilfsstoffs kann verhindert werden, daß zur Adhesion an den Wänden des Mischbehälters neigender Wirkstoff an den Wänden des Mischbehälters anhaftet (Vorbelegungseffekt durch den Hilfsstoff). Durch den Nachlauf bei der Förderung des Hilfsstoffs kann verhindert werden, daß zur Adhesion an der Siebeinrichtung neigender Wirkstoff in der Siebeinrichtung verbleibt (Spüleffekt durch den Hilfsstoff).

Je nach apparativer Ausgestaltung kann kann im Rahmen des erfindungsgemäßen Verfahrens gegebenenfalls bereits während der Förderung der einzelnen Komponenten mit dem Mischen begonnen werden. Dies ist beispielsweise möglich, wenn die Komponenten in einen feststehenden Mischbehälter gefördert werden, der sich bewegende Mischwerkzeuge aufweist. Solche Mischer sind im Stand der Technik unter der Bezeichnung Zwangsmischer bekannt.

Die vorliegende Erfindung betrifft insbesondere ein Verfahren zur Herstellung von Inhalationspulvern enthaltend weniger als 5%, bevorzugt weniger als 2%, besonders bevorzugt weniger als 1 % Wirkstoff im Gemisch mit einem physiologisch verträglichen Hilfsstoff. Erfindungsgemäß bevorzugt ist ein Verfahren zur Herstellung von Inhalationspulvern, in denen 0,04 bis 0,8%, besonders bevorzugt 0,08 bis 0,64%, besonders bevorzugt 0,16 bis 0,4% Wirkstoff im Gemisch mit einem physiologisch unbedenklichen Hilfsstoff enthalten sind.

Der verwendete Wirkstoff weist erfindungsgemäß bevorzugt eine mittlere Teilchengröße von 0,5 bis 10 μm, vorzugsweise von 1 bis 6 μm, besonders bevorzugt von 2 bis 5 μm auf. Der in das erfindungsgemäße Verfahren einsetzbare Hilfsstoff weist vorzugsweise eine mittlere Teilchengröße von 10 bis 100 μm, bevorzugt 15 bis 80 μm, besonders bevorzugt 17 bis 50 μm auf. Erfindungsgemäß besonders bevorzugt sind Verfahren zur Herstellung von Inhalationspulvern, in denen der Hilfsstoff eine mittlere Teilchengröße von 20-30 μm aufweist.

Gegebenenfalls kann der Hilfsstoff auch aus einem Gemisch von gröberem Hilfsstoff mit einer mittleren Teilchengröße von 15 bis 80μm und feinerem Hilfsstoff mit einer mittleren Teilchengröße von 1 bis 9 μm bestehen, wobei der Anteil von feinerem Hilfsstoff an der Gesamthilfsstoffmenge 1 bis 20 % betragen kann. Enthalten die mittels des erfindungsgemäßen Verfahrens herstellbaren Inhalationspulver ein Gemisch aus gröberen und feineren Hilfsstofffraktionen, so ist erfindungsgemäß die Herstellung solcher Inhalationspulver bevorzugt, in denen der gröbere Hilfsstoff eine mittlere Teilchengröße von 17 bis 50 μm, besonders bevorzugt von 20 bis 30 μm und der feinere Hilfsstoff eine mittlere Teilchengröße von 2 bis 8 μm, besonders bevorzugt von 3 bis 7 μm aufweist. Dabei wird unter der mittleren Teilchengröße im hier verwendeten Sinne der 50 %-Wert aus der Volumenverteilung gemessen mit einem Laserdiffraktometer nach der Trockendispersionsmethode verstanden. Im Fall einer Hilfsstoffmischung aus gröberen und feineren Hilfsstoffanteilen, sind erfindungsgemäß solche Verfahren bevorzugt, mit denen Inhalationspulver erhalten werden, bei denen der Anteil von feinerem Hilfsstoff an der Gesamthilfsstoffmenge 3 bis 15 %, besonders bevorzugt 5 bis 10 % beträgt.

Bei den im Rahmen der vorliegenden Erfindung genannten prozentualen Angaben, handelt es sich stets um Gewichtsprozent.

Soll als Hilfsstoff eines der vorstehend genannten Gemische von gröberem Hilfsstoff mit feinerem Hilfsstoff zum Einsatz gelangen, kann die Hilfsstoffmischung ebenfalls gemäß dem erfindungsgemäßen Verfahren erhalten werden.

In diesem Fall wird der Hilfsstoff mit der kleineren Teilchengrößenverteilung und der Hilfsstoff mit der größeren Teilchengrößenverteilung in einen geeigneten Mischbehälter kontinuierlich so eindosiert, daß der Quotient N aus der Fördergeschwindigkeit für die Zudosierung des Hilfsstoffs mit der kleineren Teilchengrößenverteilung und Fördergeschwindigeit für die Zudosierung des Hilfsstoffs mit der größeren Teilchengrößenverteilung wenigstens so groß ist wie der Quotient M aus Gesamtmasse des Hilfsstoffs mit der kleineren Teilchengrößenverteilung und Gesamtmasse des Hilfsstoffs mit der größeren Teilchengrößenverteilung. Im Falle der Herstellung einer Hilfsstoffmischung mittels des erfindungsgemäßen Herstellverfahrens ist N bevorzugt gleich M (N = M).

Aus einer so erhaltenen Hilfsstoffmischung kann anschließend mittels des erfindungsgemäßen Verfahrens das gewünschte Inhalationspulver erhalten werden. Dementsprechend betrifft die vorliegende Erfindung ferner einer Verfahren zur Herstellung von Inhalationspulvern, in dem ein Wirkstoff und eine Hilfsstoffmischung in einen geeigneten Mischbehälter kontinuierlich so eindosiert werden, daß der Quotient N aus der Fördergeschwindigkeit für die Zudosierung des Wirkstoffs und Fördergeschwindigeit für die Zudosierung der Hilfsstoffmischung wenigstens so groß ist wie der Quotient M aus Gesamtmasse des Wirkstoffs und Gesamtmasse der Hilfsstoffmischung.

Die mittels des erfindungsgemäßen Herstellverfahrens erhältlichen Inhalationspulver können generell alle Wirkstoffe enthalten, deren Applikation aus therapeutischer Sicht auf inhalativem Wege sinnvoll erscheint. Bevorzugt gelangen hierbei solche Wirkstoffe zur Anwendung, die beispielsweise ausgewählt sind aus der Gruppe bestehend aus Betamimetika, Anticholinergika, Corticosteroiden und Dopaminagonisten.

Als Betamimetika gelangen hierbei vorzugsweise Verbindungen zur Anwendung, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, Mabuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-*tert*.-butylamino)ethanol und 1-(4-Ethoxycarbonylamino-3-cyano-5-fluorophenyl)-2-(*tert*.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe zur Anwendung, die ausgewählt sind aus der Gruppe bestehend aus Fenoterol, Formoterol, Salmeterol, Mabuterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-nbutyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.

Erfindungsgemäß bevorzugt sind die Säureadditionssalze der Betamimetika ausgewählt aus der Gruppe bestehend aus Hydrochlorid, Hydrobromid, Sulfat, Phosphat, Fumarat Methansulfonat und Xinafoat. Besonders bevorzugt sind die Salze im Falle des Salmeterols ausgewählt aus Hydrochlorid, Sulfat und Xinafoat, von denen die Sulfate und Xinafoate besonders bevorzugt sind. Erfindungsgemäß von herausragender Bedeutung sind Salmeterol x ½ $H_2SO_4$ und Salmeterolxinafoat. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Als Anticholinergika gelangen in den erfindungsgemäßen Verfahren bevorzugt Salze zur Anwendung, die ausgewählt sind aus der Gruppe bestehend aus Tiotropiumsalzen, Oxitropiumsalzen und Ipratropiumsalzen, besonders bevorzugt sind dabei Tiotropium- und Ipratropiumsalze. In den vorstehend genannten Salzen stellen die Kationen Tiotropium, Oxitropium und Ipratropium die pharmakologisch wirksamen Bestandteile dar. Unter den im Rahmen der vorliegenden Erfindung einsetzbaren Salzen sind die Verbindungen zu verstehen, die neben Tiotropium, Oxitropium oder Ipratropium als Gegenion (Anion) Chlorid, Bromid, Iodid, Sulfat, Methansulfonat oder para-Toluolsulfonat enthalten. Im Rahmen der vorliegenden Erfindung sind von allen Salzen der vortshend genannten Anticholinergika das Methansulfonat, Chlorid, Bromid oder Iodid bevorzugt, wobei dem Methansulfonat oder dem Bromid besondere Bedeutung zukommt. Von erfindungsgemäß herausragender Bedeutung sind die Anticholinergika ausgewählt aus der Gruppe bestehend aus Tiotropiumbromid, Oxitropiumbromid und Ipratropiumbromid. Besonders bevorzugt ist das Tiotropiumbromid. Vorstehend genannte Anticholinergika können gegebenenfalls in Form ihrer Solvate oder Hydrate vorliegen. Im Falle des Tiotropiumbromids kommt beispielsweise dem Tiotropiumbromidmonohydrat eine erfindungsgemäß besondere Bedeutung zu.

Im Rahmen der vorliegenden Erfindung werden unter Corticosteroiden Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet: Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Das erfindungsgemäße Verfahren zur Herstellung von Pulvermischungen für die Inhalation kann zur Herstellung von Inhalationspulvern Verwendung finden, die einen oder mehrere der vorstehend genannten Wirkstoffe enthalten. Sollen beispielsweise Inhalationspulver hergestellt werden, deren pharmazeutisch aktive Bestandteile aus zwei verschiedenen Wirkstoffen bestehen, so läßt sich dies mittels des erfindungsgemäßen Verfahrens beispielsweise dadurch gewährleisten, daß zunächst zu einem in Bewegung gehaltenen Pulverbett des Hilfsstoffs oder der Hilfsstoffmischung der erste Wirkstoff kontinuierlich zudosiert wird und anschließend zu dieser Pulvermischung in analoger Weise die kontinuierliche Zudosierung des zweiten Wirkstoffs erfolgt. Soll das erfindungsgemäße Verfahren zur Herstellung von Inhalationspulvern dienen, die beispielsweise zwei Wirkstoff enthalten, so seien an dieser Stelle als mögliche Wirkstoffkombinationen die von beispielsweise einem der vorstehend genannten Anticholinergika mit einem der vorstehend genannten Corticosteroide oder die von einem der vorstehend genannten Anticholinergika mit einem der vorstehend genannten Betamimetika als bevorzugt genannt.

Als physiologisch unbedenkliche Hilfsstoffe, die im Rahmen der erfindungsgemäßen Herstellverfahren zur Anwendung gelangen können, seien beispielsweise genannt Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciulcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Inhaltionspulver, die gemäß des erfindungsgemäßen Herstellverfahrens erhalten werden können, zeichnen sich durch ein außergewöhnlich hohes Maß an Homogenität im Sinne der Gehaltsgleichförmigkeit aus. Diese liegt in einem Bereich von < 8% , bevorzugt < 6% , besonders bevorzugt < 4%. Die mittels des erfindungsgemäßen Verfahrens herstellbaren Inhalationspulver weisen gegebenenfalls gar Homogenitäten im Sinne der Einzeldosierungsgenauigkeit auf, die <3%, gegebenenfalls <2% beträgt. Somit zielt ein weiterer Aspekt der vorliegenden Erfindung auf Inhaltionspulver als solche, die mittels des erfindungsgemäßen Herstellverfahrens erhältlich sind.

WO 02/30390 (Stand der Technik gemäß Art. 54(3)/(4) EPÜ) offenbart identische Endprodukte (Pulvermischungen), die jedoch mit einem anderen Verfahren hergestellt wurden.

Die mittels des erfindungsgemäßen Verfahrens erhältlichen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die mittels des erfindungsgemäßen Verfahrens erhältlichen Inhalationspulver allerdings in Kapseln abgefüllt (zu sogenannten Inhaletten), die in Inhalatoren wie beispielsweise in der WO 94/28958 beschrieben, zur Anwendung gelangen.

Soll das mittels des erfindungsgemäßen Verfahrens erhältliche Inhalationspulver im Sinne der vorstehend gennannten bevorzugten Anwendung in Kapseln (Inhaletten) abgefüllt werden, bieten sich Füllmengen von 3 bis 10 mg, bevorzugt von 4 bis 6 mg Inhalationspulver pro Kapsel an, wobei diese Füllmenge in hohem Maße von der Wahl des verwendeten Wirkstoffs abhängig sein können. Im Falle des Wirkstoffs Tiotropiumbromid enthalten die Kapseln bei den vorstehend genannten Füllmengen zwischen 1,2 und 80 µg Tiotropiumkation. Bei einer für Tiotropiumbromid bevorzugten Füllmenge von 4 bis 6 mg Inhalationspulver pro Kapsel sind zwischen 1,6 und 48 µg, bevorzugt zwischen 3,2 und 38,4 µg, besonders bevorzugt zwischen 6,4 und 24 µg Tiotropium pro Kapsel enthalten. Ein Gehalt von beispielsweise 18 µg Tiotropium entspricht dabei einem Gehalt von etwa 21,7 µg Tiotropiumbromid.

Folglich enthalten Kapseln mit einer Füllmenge von 3 bis 10 mg Inhaltionspulver erfindungsgemäß bevorzugt zwischen 1,4 und 96,3 µg Tiotropiumbromid. Bei einer bevorzugten Füllmenge von 4 bis 6 mg Inhalationspulver pro Kapsel sind zwischen 1,9 und 57,8 µg, bevorzugt zwischen 3,9 und 46,2µg, besonders bevorzugt zwischen 7,7 und 28,9 µg Tiotropiumbromid pro Kapsel enthalten. Ein Gehalt von beispielsweise 21,7 µg Tiotropiumbromid entspricht dabei einem Gehalt von etwa 22,5 µg Tiotropiumbromid-monohydrat.

Folglich enthalten Kapseln mit einer Füllmenge von 3 bis 10 mg Inhalationspulver bevorzugt zwischen 1,5 und 100 µg Tiotropiumbromid-monohydrat. Bei einer bevorzugten Füllmenge von 4 bis 6 mg Inhalationspulver pro Kapsel sind zwischen 2 und 60 µg, bevorzugt zwischen 4 und 48 µg, besonders bevorzugt zwischen 8 und 30 µg Tiotropiumbromid-monohydrat pro Kapsel enthalten.

In den nachfolgenden Beispielen wird eine mögliche Vorgehensweise zur Durchführung des erfindungsgemäßen Verfahrens am Beispiel einer Pulvermischung enthaltend Tiotropiumbromidmonohydrat beschrieben. Die direkte Übertragbarkeit dieses bespielhaft erläuterten Verfahrens zur Herstellung von Inhalationspulvern, die einen oder mehrere der vorstehend genannten anderen Wirkstoffe enthalten, ist für den Fachmann ersichtlich. Dementsprechend dienen die folgenden Beispiele lediglich einer weitergehenden Erläuterung der vorliegenden Erfindung, ohne den Umfang der Erfindung auf die beispielhaft beschriebenen Ausführungsformen zu beschränken.

## Ausgangsmaterialien

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird als feinerer Hilfsstoff Lactose-Monohydrat (5µ) verwendet. Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

## Darstellung von Tiotropiumbromid-monohydrat:

In einem geeigneten Reaktionsgefäß werden in 25,7 kg Wasser 15,0 kg Tiotropiumbromid, welches wie in der EP 418 716 A1 offenbart, hergestellt werden kann, eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet. Ausbeute : 13,4 kg Tiotropiumbromid-monohydrat (86 % d. Th.)

Das so erhaltene kristalline Tiotropiumbromid-monohydrat wird nach bekannten Verfahren mikronisiert, um den Wirkstoff in Form der mittleren Teilchengröße bereitzustellen, die den erfindungsgemäßen Spezifikationen entspricht.

Im Sinne der vorliegenden Erfindung ist unter mittlerer Teilchengröße der Wert in µm zu verstehen, an dem 50% der Teilchen aus der Volumenverteilung eine kleinere oder die gleiche Partikelgröße besitzen im Vergleich zum angegebenen Wert. Zur Bestimmung der Summenverteilung der Partikelgrößenverteilung wird als Meßmethode Laserdiffraktion/Trockendispergierung angewendet.

Nachfolgend wird beschrieben, wie die Bestimmung der mittleren Teilchengröße der verschiedenen Bestandteile der erfindungsgemäßen Formulierung erfolgen kann.

**A) Partikelgrößenbestimmung von feinteiliger Lactose:**

Meßgerät und Einstellungen:

Die Bedienung der Geräte erfolgt in Übereinstimmung mit den Bedienungsanleitungen des Herstellers.

| Meßgerät | HELOS Laser-Beugungs-Spektrometer , (SympaTec) |
|---|---|
| Dispergiereinheit | RODOS Trockendispergierer mit Saugtrichter, (SympaTec) |
| Probenmenge | ab 100 mg |
| Produktzufuhr | Schwingrinne Vibri, Fa. Sympatec |
| Frequenz d. Vibrationsrinne | 40 bis 100 % ansteigend |
| Dauer der Probenzufuhr | 1 bis 15 sek. (im Fall von 100 mg) |
| Brennweite | 100 mm (Meßbereich: 0,9 - 175 µm) |
| Meßzeit | ca. 15 s (im Fall von 100 mg) |
| Zykluszeit | 20 ms |
| Start/Stop bei | 1 % auf Kanal 28 |
| Dispergiergas | Druckluft |
| Druck | 3 bar |
| Unterdruck | maximal |
| Auswertemodus | HRLD |

Probenvorbereitung / Produktzufuhr:

Mind. 100 mg der Prüfsubstanz werden auf einem Kartenblatt eingewogen.
Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

**B) Partikelgrößenbestimmung von Tiotropiumbromidmonohydrat, mikronisiert:**

Meßgerät und Einstellungen:

Die Bedienung der Geräte erfolgte in Übereinstimmung mit den Bedienungsanleitungen des Herstellers.

| Meßgerät | Laser-Beugungs-Spektrometer (HELOS), Sympatec |
|---|---|
| Dispergiereinheit | Trockendispergierer RODOS mit Saugtrichter,Sympatec |
| Probenmenge | 50 mg - 400 mg |
| Produktzufuhr | Schwingrinne Vibri, Fa. Sympatec |
| Frequenz d. Vibrationsrinne | 40 bis 100 % ansteigend |
| Dauer der Probenzufuhr | 15 bis 25 sek. (im Fall von 200 mg) |
| Brennweite | 100 mm (Meßbereich: 0,9 - 175 µm) |
| Meßzeit | ca. 15 s (im Fall von 200 mg) |
| Zykluszeit | 20 ms |
| Start/Stop bei | 1 % auf Kanal 28 |
| Dispergiergas | Druckluft |
| Druck | 3 bar |
| Unterdruck | maximal |
| Auswertemodus | HRLD |

Probenvorbereitung / Produktzufuhr:

ca. 200 mg der Prüfsubstanz werden auf einem Kartenblatt eingewogen.
Mit einem weiteren Kartenblatt werden alle größeren Agglomerate zerstoßen.

Das Pulver wird dann auf der vorderen Hälfte der Schwingrinne (ab ca. 1 cm vom vorderen Rand) fein verteilt aufgestreut.

Nach dem Start der Messung wird die Frequenz der Schwingrinne von ca. 40 % bis 100 % (gegen Ende der Messung) variiert. Die Zufuhr der Probe soll möglicht kontinuierlich sein. Die Produktmenge darf aber auch nicht zu groß sein damit eine ausreichende Dispergierung erreicht wird. Die Zeit, in der jeweils die gesamte Probe zugeführt wird beträgt für 200 mg z. B. ca. 15 bis 25 sek.

**C) Partikelgrößenbestimmung von Laktose 200M :**

Meßgerät und Einstellungen

Die Bedienung der Geräte erfolgte in Übereinstimmung mit den Bedienungsanleitungen des Herstellers.

| Meßgerät | Laser-Beugungs-Spektrometer (HELOS), Sympatec |
| --- | --- |
| Dispergiereinheit | Trockendispergierer RODOS mit Saugtrichter, Sympatec |
| Probenmenge | 500 mg |
| Produktzufuhr | Vibrationsrinne Typ VIBRI , Sympatec |
| Frequenz d. Vibrationsrinne | 18 bis 100 % ansteigend |
| Brennweite (1) | 200 mm (Meßbereich: 1.8 - 350 µm) |
| Brennweite (2) | 500 mm (Meßbereich: 4.5 - 875 µm) |
| Meßzeit/Wartezeit | 10 s |
| Zykluszeit | 10 ms |
| Start/Stop bei | 1 % auf Kanal 19 |
| Druck | 3 bar |
| Unterdruck | maximal |
| Auswertemodus | HRLD |

Probenvorbereitung / Produktzufuhr:

Ca. 500 mg der Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

**Apparatives**

Zur Herstellung der erfindungsgemäßen Inhalationspulver können beispielsweise die folgenden Maschinen und Geräte Verwendung finden:

Mischbehälter bzw. Pulvermischer: Rhönradmischer 200 L; Typ: DFW80N-4; Hersteller: Firma Engelsmann, D-67059 Ludwigshafen.

Siebgranulator: Quadro Comil; Typ: 197-S; Hersteller: Firma Joisten & Kettenbaum, D-51429 Bergisch-Gladbach.

Fördereinrichtung: KtronSoder; Typ T20 bzw. T35; Hersteller: Firma K-Tron Soder GmbH, D 6460 Gelnhausen.

Fördereinrichtung (für Kleinmengen/speziell Wirkstoff): Gericke; Typ GMD60/2; Hersteller: Firma Gericke GmbH, D 78239 Rielasingen.

**Beispiel 1:**

Die Durchführung des nachfolgend beschriebenen Schritts 1.1 zur Darstellung einer Hilfsstoffmischung kann gegebenenfalls auch entfallen. Wird eine Pulvermischung angestrebt, die neben dem Wirkstoff lediglich Hilfsstoff einer einheitlichen gröberen Teilchengrößenverteilung enthält, kann direkt gemäß Schritt 1.2 verfahren werden.

1.1: Hilfsstoffmischung:

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5µm) eingesetzt. In den daraus erhaltenen 33,5 kg Hilfsstoffmischung beträgt der Anteil der feineren Hilfsstoffkomponente 5%.
Über zwei getrennte geeignete Fördereinrichtungen werden 31,82 kg Lactose Monohydrat für inhalationszwecke (200M) und 1,68 kg Lactose Monohydrat (5µm) kontinuierlich in einen geeigneten Mischbehälter eindosiert. Die relative Fördergeschwindigkeit für die beiden Komponenten entspricht in etwa ihrem Anteil in der Rezeptur so daß die Zudosierung etwa gleichzeitig beendet ist (z.B. ca. 950 g/min für Lactose Monohydrat für Inhalationszwecke (200M) und ca. 50 g/min für Lactose Monohydrat (5µm)). Bei Bedarf kann die Zudosierung der beiden Komponenten in den Mischbehälter über einen geeigneten Siebgranulator mit einem Sieb mit einer Maschenweite von 0,5 mm erfolgen.
Die eingesiebten Bestandteile werden anschließend gemischt (Mischen: 900 Umdrehungen)

1.2: wirkstoffhaltige Pulvermischung:

Zur Herstellung der Endmischung werden 32,87 kg der Hilfsstoffmischung (1.1) und 0,13 kg Mikronisiertes Tiotropiumbromid-monohydrat eingesetzt. In den daraus erhaltenen 33,0 kg Inhalationspulver beträgt der Wirkstoffanteil 0.4 %.
Über zwei getrennte geeignete Fördereinrichtungen werden 32,87 kg Hilfsstoffmischung (1.1) und 0,13 kg mikronisiertes Tiotropiumbromid-monohydrat kontinuierlich in einen geeigneten Mischbehälter eindosiert. Die relative Fördergeschwindigkeit für die Hilfsstoffmischung (1.1) ist dabei im Verhältnis zu der Fördergeschwindigkeit des Wirkstoffes etwas geringer als es den Anteilen in der Rezeptur entspricht wobei die Zudosierung der Hilfsstoffmischung etwas früher gestartet wird und nach vollständiger Zugabe des Wirkstoffes noch etwas länger gefördert wird (z.B. ca. 950 g/min für die Hilfsstoffmischung und ca. 4 g/min für Tiotropiumbromid-monohydrat). Bei Bedarf kann die Zudosierung der beiden Komponenten in den Mischbehälter über einen geeigneten Siebgranulator mit einem Sieb mit einer Maschenweite von 0,5 mm erfolgen. Durch den Vorlauf / Nachlauf bei der Förderung der Hilfsstoffmischung wird dabei die im Siebgranulator verbleibende Wirkstoffmenge minimiert (Vorbelegungs- und Spüleffekt).
Die eingesiebten Bestandteile werden anschließend gemischt (Mischen: 900 Umdrehungen). Die Endmischung wird noch zweimal über einen Siebgranulator gegeben und anschließend jeweils gemischt (Mischen: 900 Umdrehungen).

**Beispiel 2:**

Mit der nach Beispiel 1 erhaltenen Mischung werden Inhalationskapseln (Inhaletten) der folgenden Zusammensetzung erhalten:

| Tiotropiumbromid-monohydrat | 0,0225 mg |
|---|---|
| Lactose Monohydrat (200 M) | 5,2025 mg |
| Lactose Monohydrat (5 µm) | 0,2750 mg |
| Hartgelatinekapsel | 49,0 mg |
| Total | 54,5 mg |

**Beispiel 3:**

Inhalationskapsel der Zusammensetzung:

| Tiotropiumbromid-monohydrat | 0,0225 mg |
|---|---|
| Lactose Monohydrat (200 M) | 4,9275 mg |
| Lactose Monohydrat (5 µm) | 0,5500 mg |
| Hartgelatinekapsel | 49,0 mg |
| Total | 54,5 mg |

Das zur Herstellung der Kapsel erforderliche Inhalationspulver wurde in Analogie zu Beispiel 1 erhalten.

**Beispiel 4:**

Inhalationskapsel der Zusammensetzung:

| | |
|---|---|
| Tiotropiumbromid-monohydrat | 0,0225 mg |
| Lactose Monohydrat (200 M) | 5,2025 mg |
| Lactose Monohydrat (5 μm) | 0,2750 mg |
| Polyethylenkapsel | 100,0 mg |
| Total | 105,50 mg |

Das zur Herstellung der Kapsel erforderliche Inhalationspulver wurde in Analogie zu Beispiel 1 erhalten.

**Patentansprüche**

1. Verfahren zur Herstellung von Inhalationspulvern, **dadurch gekennzeichnet, daß** eine Substanz mit kleinerer Teilchengrößenverteilung und eine Substanz mit größerer Teilchengrößenverteilung in einen geeigneten Mischbehälter kontinuierlich so eindosiert werden, daß der Quotient N aus der Fördergeschwindigkeit für die Zudosierung der Substanz mit der kleineren Teilchengrößenverteilung und Fördergeschwindigeit für die Zudosierung der Substanz mit der größeren Teilchengrößenverteilung wenigstens so groß ist wie der Quotient M aus Gesamtmasse der Substanz mit der kleineren Teilchengrößenverteilung und Gesamtmasse der Substanz mit der größeren Teilchengrößenverteilung.

2. Verfahren nach Anspruch 1, **dadurch gekennzeichnet, daß** der Quotient N größer als der Quotient M ist.

3. Verfahren nach Anspruch 1 oder 2, **dadurch gekennzeichnet, daß** der Quotient N/M in einem Bereich von $1 < N/M \leq 1,5$ liegt.

4. Verfahren nach Anspruch 1, 2 oder 3, **dadurch gekennzeichnet, daß** die Zugabe der Komponenten der Pulvermischung über eine geeignete Siebeinrichtung erfolgt.

5. Verfahren nach einem der Ansprüche 1 bis 4, **dadurch gekennzeichnet, daß** Pulvermischungen für die Inhalation erzeugt werden, in der der Gehalt der Substanz mit der kleineren Teilchengrößenverteilung bei weniger als 5% liegt.

6. Verfahren nach einem der Ansprüche 1 bis 5, **dadurch gekennzeichnet, daß** Pulvermischungen für die Inhalation erzeugt werden, in der die Substanz mit der kleineren Teilchengrößenverteilung eine mittlere Teilchengröße von 0,5 bis 10 μm und in der die Substanz mit der größeren Teilchengrößenverteilung eine mittlere Teilchengröße von 10 bis 100 μm aufweist.

7. Verfahren nach einem der Ansprüche 1 bis 6, **dadurch gekennzeichnet, daß** Pulvermischungen für die Inhalation erzeugt werden, in der die Substanz mit der kleineren Teilchengrößenverteilung ausgewählt ist aus der Gruppe bestehend aus Betamimetika, Anticholinergika, Corticosteroiden und Dopaminagonisten.

**Claims**

1. Process for preparing inhalable powders, **characterised in that** a substance with a smaller particle size distribution and a substance with a coarser particle size distribution are continuously metered into a suitable mixing container in such a way that the quotient N of the delivery speed for the metering of the substance with the smaller particle size distribution and the delivery speed for the metering of the substance with the larger particle size distribution is at least as great as the quotient M of the total mass of the substance with the smaller particle size distribution and the total mass of the substance with the larger particle size distribution.

2. Process according to claim 1, **characterised in that** the quotient N is greater than the quotient M.

3. Process according to claim 1 or 2, **characterised in that** the quotient N/M is in the range from $1 < N/M \leq 1.5$.

4. Process according to one of claims 1, 2 or 3, **characterised in that** the components of the powder mixture are added by means of a suitable screening device.

5. Process according to one of claims 1 to 4, **characterised in that** powder mixtures for inhalation are produced wherein the content of the substance with the smaller particle size distribution is less than 5%.

6. Process according to one of claims 1 to 5, **characterised in that** powder mixtures for inhalation are produced wherein the substance with the smaller particle size distribution has a mean particle size of from 0.5 to 10 µm and wherein the substance with the larger particle size distribution has a mean particle size of from 10 to 100 µm.

7. Process according to one of claims 1 to 6, **characterised in that** powder mixtures for inhalation are produced wherein the substance with the smaller particle size distribution is selected from among the betamimetics, anticholinergics, corticosteroids and dopamine agonists.


**Revendications**

1. Procédé de production de poudres pour inhalation **caractérisé en ce qu'**une substance de répartition granulométrique plus petite et une substance de répartition granulométrique plus grande sont dosées en continu dans un récipient mélangeur approprié de telle manière que le quotient N de la vitesse de transport pour le dosage de la substance de répartition granulométrique plus petite et de la vitesse de transport pour le dosage de la substance de répartition granulométrique plus grande est au moins aussi grand que le quotient M de la masse totale de la substance de répartition granulométrique plus petite et de la masse totale de la substance de répartition granulométrique plus grande.

2. Procédé selon la revendication 1 **caractérisé en ce que** le quotient N est supérieur au quotient M.

3. Procédé selon la revendication 1 ou 2 **caractérisé en ce que** le quotient N/M est situé dans une plage de $1 < N/M \le 1,5$.

4. Procédé selon la revendication 1, 2 ou 3 **caractérisé en ce que** l'addition des composants du mélange pulvérulent a lieu par le biais d'un dispositif de tamisage approprié.

5. Procédé selon l'une des revendications 1 à 4 **caractérisé en ce que** des mélanges pulvérulents dans lesquels la teneur de la substance de répartition granulométrique plus petite est située à moins de 5 % sont produits.

6. Procédé selon l'une des revendications 1 à 5 **caractérisé en ce que** des mélanges pulvérulents pour l'inhalation dans lesquels la substance de répartition granulométrique plus petite présente une taille particulaire moyenne de 0,5 à 10 µm et dans lesquels la substance de répartition granulométrique plus grande présente une taille particulaire moyenne de 10 à 100 µm sont produits.

7. Procédé selon l'une des revendications 1 à 6 **caractérisé en ce que** des mélanges pulvérulents pour l'inhalation dans lesquels la substance de répartition granulométrique plus petite est choisie dans le groupe consistant en les bêta-mimétiques, les anticholinergiques, les corticostéroïdes et les agonistes de dopamine sont produits.